# EUROPEAN PATENT SPECIFICATION

(11) **EP 3 422 004 B1**
(45) Date of publication and mention of the grant of the patent: **09.09.2020**
(21) Application number: 17756565.2
(22) Date of filing: 23.02.2017
(51) Int. Cl.: G01N 33/574, G01N 33/543, G01N 33/553, G01N 33/577, G01N 33/558, G01N 33/68

(54) **METHOD FOR DETECTING CARCINOEMBRYONIC FIBRONECTIN USING IMMUNOCHROMATOGRAPHY**
VERFAHREN ZUM NACHWEIS VON KARZINOEMBRYNALEM FIBRONECTIN MITHILFE VON IMMUNCHROMATOGRAFIE
PROCÉDÉ DE DÉTECTION DE FIBRONECTINE CARCINOEMBRYONNAIRE PAR IMMUNOCHROMATOGRAPHIE

(30) Priority: 24.02.2016 JP 2016033463
(43) Date of publication of application: 02.01.2019
(73) Proprietor: Sekisui Medical Co., Ltd., Tokyo 103-0027 (JP)
(72) Inventor: ITOU, Kanako, Tokyo 103-0027 (JP); MORITA, Motoki, Tokyo 103-0027 (JP); KOHNO, Keigo, Tokyo 103-0027 (JP)
(74) Representative: Hoffmann Eitle
(86) International application number: PCT/JP2017/006728
(87) International publication number: WO 2017/146127

(56) References cited:
- EP-A1- 2 835 643
- WO-A1-92/10585
- WO-A1-99/39298
- JP-A- H01 195 848
- JP-A- 2002 502 045
- JP-A- 2011 039 068
- JP-A- 2013 539 041
- US-A1- 2006 024 725
- IAMS, JAY D. ET AL.: 'Fetal Fibronectin Improves the Accuracy of Diagnosis of Preterm Labor' AMERICAN JOURNAL OF OBSTETRICS & GYNECOLOGY vol. 173, no. 1, 1995, pages 141 - 145, XP002379904
- LOCKWOOD, CHARLES J. ET AL.: 'Fetal Fibronectin in Cervical and Vaginal Secretions as a Predictor of Preterm Delivery' THE NEW ENGLAND JOURNAL OF MEDICINE vol. 325, no. 10, 05 September 1991, pages 669 - 674, XP055412099
- MATSUURA, HIDEMITSU ET AL.: 'The Oncofetal Domain of Fibronectin Defined by Monoclonal Antibody FDC-6: Its Presence in Fibronectins from Fetal and Tumor Tissues and Its Absence in Those from Normal Adult Tissues and Plasma' PROCEEDINGS OF THE NATIONAL ACADEMY OF SCIENCES vol. 82, October 1985, pages 6517 - 6521, XP000984048

## Description

### TECHNICAL FIELD

The present invention relates to a detection method using immunochromatography in the case where an analyte and a substance having antigenicity similar thereto may coexist in a sample. More particularly, the present invention relates to a method of detecting oncofetal fibronectin using immunochromatography in the case where other fibronectins may coexist.

### BACKGROUND ART

Point of Care Testing (POCT) is a collective term for tests generally conducted near a patient, such as a general practitioner, a hospital ward, or a clinic for outpatient treatment, etc. Since diagnosis and treatment may be quickly performed according to test results obtained by a simple manipulation, it is expected to contribute to the quality improvement of medical treatment.

A detection method using immunochromatography (hereinafter, simply referred to as an immunochromatographic method) which is a method frequently used in POCT is practically used in a variety of fields, including infectious diseases such as influenza infection, obstetrics such as pregnancy, preterm birth, etc., lifestyle diseases such as diabetes, heart diseases, etc.

A heterogeneous method includes a step (hereinafter, also simply referred to as a washing step) of washing components in sample other than an antigen-antibody complex derived from an analyte prior to detection of the analyte during the operation (hereinafter, also simply referred to as a heterogeneous method). On the other hand, a homogeneous method does not include the corresponding washing step (hereinafter, also simply referred to as homogeneous method) . The immunochromatographic method corresponds to a homogeneous method.

In the case where a substance having antigenicity similar to an analyte (hereinafter, also referred to as a similar antigen) exists in a sample (specimen), the substance may cause an error to a measurement result in the homogeneous method, even if it exists in an amount that does not cause problems in the heterogeneous method.

An analyte possibly containing similar antigens due to contamination of a sample with blood or the like may be exemplified by fibronectin. Fibronectins constitute a family of proteins expressed from a single gene. It is known that various isoforms of fibronectin are present in plasma and living tissues, including connective tissue, skin, colon, liver, spleen, and kidney (Matsuura and Hakomori, Proc. Natl. Acad. Sci. USA 82: 6517-6521 (1985)). Fetal tissues and several different kinds of tumor cells include fibronectin isoforms collectively called "fetal" or "oncofetal" fibronectins. Oncofetal fibronectin (hereinafter, also simply referred to as "fFN") is rarely present in vaginal secretions of non-pregnant women or pregnant women after 22 weeks of gestation who have no disorders in the fetal membrane, but oncofetal fibronectin leaks into the vaginal secretions when the fetal membrane is damaged or weakened by bacterial infections or physical factors. Therefore, fFN concentrations in the vaginal secretions of pregnant women at 22 weeks to 33 weeks of gestation are useful as a marker for threatened premature labor. Such an oncofetal fibronectin tested in obstetrics is initially written as "oncofetal fibronectin", and in Japanese, it has been translated and used as "Gantaijisei" fibronectin. Currently, an expression of "fetal" fibronectin tends to be used. Unless otherwise specified in the present invention, the terms are unified by the expression of "oncofetal fibronectin".

As described above, fibronectin is a family of proteins expressed from a single gene and is a major protein with a molecular weight of about 500 kDa which constitutes the extracellular matrix. fFN has the same amino acid sequence as plasma-derived FN (hereinafter, also abbreviated to plasma FN or FN), and they are different from each other only in the sugar chain binding site. fFN has a sugar content of about 9.5% and plasma fibronectin has a sugar content of 5.8%. As described above, since plasma FN and fFN have the same amino acid sequence and there is only one fFN-specific epitope currently identified, it is very difficult to obtain two or more kinds of antibodies that specifically react with fFN, but completely do not react with plasma FN.

Therefore, as two antibodies to form a sandwich complex, a pair of antibodies may be needed, one antibody that specifically binds to fFN but does not bind to the other fibronectin and the other antibody that binds to both fibronectins.

Practically, in order to obtain a monoclonal antibody that specifically binds to fFN, the applicant attempted to obtain the monoclonal antibody by using a peptide containing a sugar chain moiety peculiar to fFN as an immunogen, and as a result, the obtained monoclonal antibody was an antibody that specifically reacts with fFN, but shows a slight cross-reactivity with plasma FN.

As a reagent that is currently being distributed as an *in vitro* diagnostic medical product of fFN, "immunotester (registered trademark) fFN" (manufactured by Sekisui Medical Co., Ltd.) which is a reagent employing an ELISA method is known (Non-Patent Document 1).

The immunotester employs a two-step sandwich ELISA method using an immunoplate and employs a heterogeneous method including a washing step. This reagent has a cut-off value of 50 ng/mL, based on the risk of threatened premature labor. Even though a specimen is contaminated with blood, false positive reactions hardly occur and no clinical problems are caused, because of the washing step.

Meanwhile, since a detection method using immunochromatography is a homogeneous method including no washing step, there is a problem that this method is vulnerable to blood contamination, etc. For example, a specimen with blood contamination of 0.1% or more may be distinguished by the color, but it is difficult to visually distinguish a specimen with blood contamination of less than 0.1%. Therefore, a reagent which does not cause a false positive reaction even when blood contamination is less than 0.1% is desirable.

As a method of detecting human fFN using immunochromatography, those in Patent Documents 1, 2 and 3 are known. However, there are no commercialized reagents that specifically detect oncofetal fibronectin. Patent Document 1 is a document that discloses general immunochromatography, and this document merely mentions oncofetal fibronectin as an analyte. Patent Document 2 discloses addition of BSA, etc. to a sample in order to inhibit non-specific binding of anti-fFN antibody with a background material.

Further, there is no description or suggestion of problems associated with antigen similarity between fFN and other fibronectins in any of Patent Documents 1 and 2.

### CITATION LIST

### PATENT LITERATURE

Patent Document 1: Japanese Patent Application Laid-Open No. 2008-514900
Patent Document 2: Japanese Patent Application Laid-Open No. 2011-39068
Patent Document 3 : Wo 1999 / 039298

### NON-PATENT LITERATURE

Non-Patent Document 1: Attached document for *in vitro* diagnostic medical product, "immunotester (registered trademark) fFN" (Sekisui Medical Co., Ltd.)

### SUMMARY OF INVENTION

### TECHNICAL PROBLEM

When an analyte in a sample possibly contaminated with a similar antigen is detected using immunochromatography, the similar antigen may be also detected, which may cause a problem of obtaining measurement value errors or false positive results.

In particular, when fFN in a sample possibly contaminated with blood is detected using immunochromatography, plasma-derived fibronectin is also detected, which may cause a problem of obtaining measurement value errors or false positive results.

In order to solve the above problem, an object of the present invention is to provide a detection method using immunochromatography, which suppresses cross-reaction with antigenic components similar to an analyte, reduces detection of similar antigens, and prevents measurement value errors and false positive reactions, while maintaining or improving the detection sensitivity for the analyte and allowing accurate determinations.

Particularly, an object of the present invention is to provide a method of detecting fFN using immunochromatography, which prevents measurement errors and false positive reactions even when a specimen is contaminated with blood, maintains or improves the detection sensitivity for fFN, and allows accurate determinations.

### SOLUTION TO PROBLEM

As a result of intensive studies to solve the above problems, it was found that when an analyte is allowed to react with an antibody specifically binding to the analyte under a condition of using a buffer solution in a specific range of pH, even though similar antigens coexist, cross-reactivity between the antibody and similar antigens may be suppressed and the analyte may be accurately detected, thereby completing the present invention.

Specifically, the present invention is as follows:
<1> A detection method using immunochromatography, comprising steps (A) to (C):
   (A) supplying a sample possibly containing an analyte and a substance similar to the analyte to a sample-supplying portion of the following test strip;
      the test strip comprising a membrane consisting of a porous body equipped with at least the sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-analyte antibody and the other is an antibody binding to the analyte, and wherein the antibody binding to the analyte may be any antibody binding to the analyte and any antibody binding to an antigen similar to the analyte;
   (B) bringing the analyte in the sample into contact with the conjugate in the presence of a buffer solution of pH 5.0 to pH 6.7; and
   (C) detecting a complex of the analyte in the sample and the conjugate in the detecting portion;
      wherein the analyte is oncofetal fibronectin, and the substance similar to the analyte is plasma fibronectin.
<3> The detection method of <2>, wherein the first antibody is an anti-fibronectin monoclonal antibody, and the second antibody is an anti-oncofetal fibronectin monoclonal antibody.
<4> The detection method of any one of <1> to <3>, wherein the buffer solution is any one or more selected from the group consisting of phosphate, ADA, MES, ACES, Bis-Tris, citrate, HEPES, MOPS, PIPES, and Tris buffer solution.
<5> The detection method of any one of <1> to <4>, wherein a concentration of the buffer solution is 20 mmol/L to 80 mmol/L.
<6> The detection method of any one of <1> to <5>, wherein the labeling substance is colloidal gold.
<7> An immunochromatographic test strip comprising the following components (1) and (2):
   (1) a membrane consisting of a porous body equipped with at least a sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-analyte antibody and the other is an antibody binding to the analyte, and wherein the antibody binding to the analyte may be any antibody binding to the analyte and any antibody binding to an antigen similar to the analyte;
   (2) a buffer solution component of pH 5.0 to pH 6.7 which is contained in at least a part of a portion from the sample-supplying portion to the detecting portion of the spreading portion;
      wherein the analyte is oncofetal fibronectin and the substance similar to the analyte is plasma fibronectin.
<8> The test strip of <7>, wherein a sample pad, a conjugate pad, and an antibody-immobilized membrane are arranged in this order from the upstream, the sample pad includes the sample-supplying portion, the conjugate pad includes the conjugate-retaining part and the buffer solution component, and the antibody-immobilized membrane includes the detecting portion.
<9> The test strip of <7> or <8>, further comprising a third pad between the conjugate pad and the antibody-immobilized membrane.
<10> The test strip of any one of <7> to <9>, wherein the analyte is oncofetal fibronectin, the substance similar to the analyte is plasma fibronectin, any one of the first antibody and the second antibody is an anti-fibronectin monoclonal antibody, and the other is an anti-oncofetal fibronectin monoclonal antibody.
<11> The test strip of <10>, wherein the first antibody is an anti-fibronectin monoclonal antibody, and the second antibody is an anti-oncofetal fibronectin monoclonal antibody.
<12> The test strip of any one of <7> to <11>, wherein the buffer solution is any one or more selected from the group consisting of phosphate, ADA, MES, ACES, Bis-Tris, citrate, HEPES, MOPS, PIPES, and Tris buffer solution.
<13> The test strip of any one of <7> to <12>, wherein a concentration of the buffer solution is 20 mmol/L to 80 mmol/L.
<14> The test strip of any one of <7> to <13>, wherein the labeling substance is colloidal gold.
<15> An immunochromatographic detection kit comprising the following components (1) and (2):
   (1) an immunochromatographic test strip comprising a membrane consisting of a porous body equipped with at least a sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-analyte antibody and the other is an antibody binding to the analyte, and wherein the antibody binding to the analyte may be any antibody binding to the analyte and any antibody binding to an antigen similar to the analyte;
   (2) a buffer solution of pH 5.0 to pH 6.7; wherein the analyte is oncofetal fibronectin, the substance similar to the analyte is plasma fibronectin.
<16> The detection kit of <15>, wherein the analyte is oncofetal fibronectin, the substance similar to the analyte is plasma fibronectin, any one of the first antibody and the second antibody is an anti-fibronectin monoclonal antibody, and the other is an anti-oncofetal fibronectin monoclonal antibody.
<17> The detection kit of <15> or <16>, wherein the buffer solution is used as a spreading solution or a specimen extraction solution.
<18> The detection kit of any one of <15> to <17>, wherein the buffer solution is any one or more selected from the group consisting of phosphate, ADA, MES, ACES, Bis-Tris, citrate, HEPES, MOPS, PIPES, and Tris buffer solution.
<19> The detection kit of any one of <15> to <18>, wherein a concentration of the buffer solution is 20 mmol/L to 80 mmol/L.
<20> A method of reducing cross-reaction between an analyte and a substance similar to the analyte, in a method of detecting an analyte in a sample using an immunochromatographic test strip,
   the method of reducing cross-reaction comprising the following steps:
   (A) supplying a sample possibly containing the analyte and the substance similar to the analyte to a sample-supplying portion of the following test strip;
      the test strip comprising a membrane consisting of a porous body equipped with at least a sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-analyte antibody and the other is an antibody binding to the analyte, and wherein the antibody binding to the analyte may be any antibody binding to the analyte and any antibody binding to an antigen similar to the analyte;
   (B) bringing the analyte in the sample into contact with the conjugate in the presence of a buffer solution of pH 5.0 to pH 6.7; and
   (C) detecting a complex of the analyte in the sample and the conjugate in the detecting portion;
      wherein the analyte is oncofetal fibronectin, and the substance similar to the analyte is plasma fibronectin.
<22> The method of <21>, wherein the first antibody is an anti-fibronectin monoclonal antibody, and the second antibody is an anti-oncofetal fibronectin monoclonal antibody.
<23> The method of any one of <20> to <22>, wherein the buffer solution is any one or more selected from the group consisting of phosphate, ADA, MES, ACES, Bis-Tris, citrate, HEPES, MOPS, PIPES, and Tris buffer solution.
<24> The method of any one of <20> to <23>, wherein a concentration of the buffer solution is 20 mmol/L to 80 mmol/L.
<25> The method of any one of <20> to <24>, wherein the labeling substance is colloidal gold.
<26> A spreading solution for immunochromatography for detecting oncofetal fibronectin, the spreading solution comprising the following (1) and (2):
   (1) a buffer solution of pH 5.0 to pH 6.7, and
   (2) a conjugate containing a first antibody labeled with a labeling substance.
<27> The spreading solution of <26>, wherein a concentration of the buffer solution is 20 mmol/L to 80 mmol/L.
<28> Use of a specimen extraction solution comprising a buffer solution of pH 5.0 to pH 6.7 for detecting oncofetal fibronectin.

### ADVANTAGEOUS EFFECTS OF INVENTION

In an immunochromatographic method according to the present invention, even when similar antigens are present, cross-reactions between an antibody against an analyte and the similar antigens are suppressed and measurement value errors and false positive reactions are prevented, while the detection sensitivity for the analyte is maintained or improved and accurate determinations are allowed.

Particularly, in a method of detecting oncofetal fibronectin (fFN) using immunochromatography, even when a specimen is contaminated with blood, measurement errors and false positive reactions hardly occur and the detection sensitivity for fFN is maintained or improved and accurate determinations are allowed.

### BRIEF DESCRIPTION OF DRAWINGS

FIG. 1 shows a schematic structure of an exemplary immunochromatographic test strip of the present invention;
FIG. 2 is a graph showing experimental results of examining effects of pH difference on cross-reactivity with plasma fibronectin (Example 1, pH 5.5, 6.0, 7.0);
FIG. 3 is a graph showing experimental results of examining effects of concentration difference of a phosphate buffer (pH 6.0) on cross-reactivity with plasma fibronectin (Example 2);
FIG. 4 is a graph showing experimental results of examining effects of concentration and pH differences of phosphate buffer on cross-reactivity with plasma fibronectin (Example 3);
FIG. 5 is a graph showing experimental results of examining effects of concentration and pH differences of ADA buffer on cross-reactivity with plasma fibronectin (Example 3); and
FIG. 6 is a graph showing experimental results of examining effects of concentration and pH differences of MES buffer on cross-reactivity with plasma fibronectin (Example 3) .

### DESCRIPTION OF EMBODIMENTS

In the present invention, the "sample" may be any of urine, lymph, blood, plasma, serum, saliva, seminal fluid, lavages, cervical fluid, cervicovaginal fluid, vaginal fluid, breast fluid, breast milk, synovial fluid, semen, seminal fluid, stool, sputum, cerebral spinal fluid, tears, mucus, interstitial fluid, follicular fluid, amniotic fluid, aqueous humor, vitreous humor, peritoneal fluid, ascites, sweat, lymphatic fluid, and lung sputum.

A component separated and fractionated from the specimen by means of centrifugation, filtration, purification, etc., a component extracted with an organic solvent, etc., a component solubilized by a surfactant, etc., a component diluted with a buffer solution, etc., a component modified/altered by chemical reaction, etc. are also included in the specimen of the present invention.

In the present specification, the "specimen" is used as a synonym for "sample", and is a liquid (fluid) when it is added and spread to the solid phase of the immunochromatography of the present invention.

The analyte of the present invention is a component contained in the "sample", for example, coagulation or fibrinolysis markers such as fibronectin, fFN, fibrin degradation products (e.g., D-dimer), soluble fibrin, TAT (thrombin-antithrombin complex), PIC (plasmin-plasmin inhibitor complex), etc., circulation-related markers such as oxidized LDL, BNP (brain natriuretic peptide), etc., metabolism-related markers such as adiponectin, etc., tumor markers such as CEA (carcinoembryonic antigen), AFP (α-fetoprotein), CA19-9, CA125, PSA (prostate-specific antigen), etc., inflammation-related markers such as CRP (C-reactive protein), IgA, IgG, IgM, etc., infection-related markers such as influenza, HIV (human immunodeficiency virus), HBV (hepatitis B virus), HCV (hepatitis C virus), toxoplasma, chlamydia, syphilis, etc., allergen-specific IgE (immunoglobulin E), hormones, drugs, nucleic acid chains and fragments thereof that involve single nucleotide polymorphisms (SNPs), and the complementary strands of the nucleic acid chains.

Among them, the analyte is preferably an antigen, which may exist in a sample, together with an antigen similar to the analyte. The analyte is preferably an antigen whose amino acid sequence is identical or has very high identity (80% or more, preferably 90% or more, and more preferably 95% or more) to an amino acid sequence of an antigen similar to the analyte. As the antigen, fibronectin having a protein family is preferred, and oncofetal fibronectin (fFN) is the most preferred analyte of the present invention.

### (Specific Buffer Solution)

In the detection method using immunochromatography, the buffer solution of the present invention is required to have actions to reduce cross-reaction between an antibody against an analyte and an antigen similar to the analyte even in the presence of the similar antigen and to maintain or improve the detection sensitivity for the analyte.

For example, when the analyte is fFN and the similar antigen is plasma FN, the buffer solution of the present invention is required to have actions to suppress cross-reactivity between plasma FN in the sample and anti-fFN antibody and to maintain or improve the detection sensitivity for fFN.

The buffer solution having these actions may be a buffer solution having pH of 5.0 to 6.7, preferably a buffer solution having pH of 5.0 to 6.5, more preferably a buffer solution having pH of 5.5 to 6.5, and much more preferably a buffer solution having pH of 5.5 to 6.0. It is not known that these buffer solutions maintain or improve a reaction in which a specific antibody bonds to only one of two kinds of antigens having similar antigenicity and suppress a cross-reaction with the other antigen.

Examples of the buffer solution include phosphate, ADA, MES, ACES, Bis-Tris, citrate, MOPS, PIPES and Tris buffer solution. Among them, phosphate, ADA, MES, Bis-Tris, and citrate buffer solutions are preferred.

A concentration of the buffer solution component in the buffer solution may be any amount as long as it is able to suppress cross-reactions other than the reaction between the analyte and the specific antibody thereto, and for example, the concentration may be 10 mmol/L or more, preferably in the range of 10 mmol/L to 100 mmol/L, 10 mmol/L to 90 mmol/L, 20 mmol/L to 80 mmol/L, 20 mmol/L to 70 mmol/L, 20 mmol/L to 60 mmol/L, 20 mmol/L to 50 mmol/L, 20 mmol/L to 40 mmol/L in a solution which is impregnated in one or both of the sample pad and the conjugate pad.

As a use mode of the specific buffer solution of the present invention, the specific buffer solution may be preferably impregnated into at least a portion of the immunochromatographic test strip from the sample-supplying portion to the detecting portion of the spreading portion to allow contact with the specimen components, and more preferably, from the sample-supplying portion to the conjugate-immobilized part of the spreading portion. Therefore, the specific buffer solution may be impregnated into only the sample-supplying portion or into the entire part of from the sample-supplying portion to the spreading portion. The mode of the sample-supplying portion and/or the conjugate pad impregnated with the buffer solution includes a mode in which pads are impregnated with a liquid buffer solution as well as a mode in which pads are dried after the pads are impregnated with the buffer solution such that the buffer solution component is attached to the pads in a dry state.

The specific buffer solution of the present invention may be also used as a specimen extraction solution. In this case, the concentration of the buffer solution is preferably 10 mmol/L or more, preferably 20 mmol/L to 80 mmol/L in the specimen extraction solution. Effective pH is 5.0 to 6.7, preferably 5.0 to 6.5, more preferably 5.5 to 6.5, and much more preferably 5.5 to 6.0 in the specimen extraction solution.

In the present invention, although the buffer solution must be used in a specific range of pH as described above, this does not prevent use of another buffer solution other than those described above for another purpose, or use of the buffer solution in another use mode other than those described above. For example, the specific buffer solution of the present invention is impregnated into from the sample-supplying portion to a conjugate-immobilized part of the spreading portion while another buffer solution other than those described above is impregnated into an antibody-immobilized part of the spreading portion, and this mode is obviously included within the scope of the present invention.

### (Immunochromatographic Test Strip)

The immunochromatographic test strip of the present invention is a membrane consisting of a porous body equipped with at least a "sample-supplying portion", a "spreading portion", and a "detecting portion", and has a structure in which a labeled antibody against an analyte is retained at a spreading start part of the spreading portion in a dissoluble manner such that the labeled antibody passes through the spreading portion and reaches the detecting portion after contact with a sample, while an immobilized antibody is immobilized at a part of the spreading portion to constitute the detecting portion.

An example of embodying these elements may be a test strip including a sample pad playing a role of a sample-supplying portion, a conjugate pad having a labeled antibody against an analyte retained in a dissoluble manner and playing a role of the spreading portion, and an insoluble membrane having an immobilized antibody immobilized at a part thereof and playing a role of the spreading portion and the detecting portion. That is, a typical immunochromatographic test strip of the present invention has the following configuration:
(1) a sample pad to which a sample is supplied;
(2) a conjugate pad which is disposed in the downstream of the sample pad and retains a conjugate in a dissoluble manner, the conjugate having a first antibody sensitized on a colloidal gold surface; and
(3) an insoluble membrane to which a second antibody binding to a complex of the conjugate and the analyte is immobilized, and the membrane being disposed in the downstream of the conjugate pad,
   wherein the sample pad, the conjugate pad, and the insoluble membrane may constitute respective different carriers, or two of the elements may constitute one carrier, and any mode may be taken as long as the sample pad, the conjugate pad, and the insoluble membrane are arranged in this order from the upstream toward the downstream of the flow of the sample.

The immunochromatographic test strip may be a strip further having any one or more of an absorption pad and a third pad disposed and mounted along with the above constituents. The test strip is usually disposed on a solid phase support such as a plastic adhesive sheet. It is obvious that the solid phase support is made of a material not hindering the capillary flow of the sample and that an adhesive component is made of a material not hindering the capillary flow of the sample. Further, the test strip may be laminated with a polyester film, etc., for the purpose of increasing the mechanical strength of the antibody-immobilized membrane and preventing evaporation (drying) of water during an assay.

### (Label)

As a "label" used in the present invention, visualizable microparticles such as metal colloid particles (colloidal gold, etc.) and colored particles (colored latex particles, etc.), magnetic particles, fluorescent particles, enzymes such as horseradish peroxidase, alkaline phosphatase, and β-D-galactosidase, etc., radioisotopes such as iodine-125, etc., luminescent substances such as acridinium compounds, luminol, etc., fluorescent substances such as fluorescein isothiocyanate, europium (III) chelate, etc. are known. The metal colloid and colored latex are preferred. As a method of labeling an antibody, etc. (i.e., a method of introducing and binding a labeling substance), any known method may be used without limitation.

Colloidal gold may be any colloidal gold as long as it is able to form a conjugate through sensitization with (immobilization of) an antibody and to serve as a label in a method of detecting a target object (antigen) in a sample through contact with the sample.

As the colloidal gold, colloidal platinum as well as colloidal gold is included in the colloidal gold of the present invention.

A particle diameter of colloidal gold particles is known to significantly affect the detection sensitivity, and for example, when the colloidal gold particles are retained and used in an immunochromatographic test strip, the particle diameter of colloidal gold particles is preferably 20 nm to 60 nm, more preferably 40 nm to 60 nm, and particularly preferably 45 nm to 55 nm. The colloidal gold may be manufactured by a generally known method, for example, by dropping and stirring a trisodium citrate aqueous solution or a triammonium citrate aqueous solution in a heated tetrachloroauric(III) acid aqueous solution.

### (Sensitization of Antibody with Colloidal Gold)

Immobilization of antibody against an analyte onto colloidal gold is generally achieved by physical adsorption. In this regard, a concentration of the antibody is preferably adjusted to a concentration of 1 µg/mL to 5 µg/mL buffer solution. A type and pH of the buffer solution are preferably 2 to 10 mmol/L tris buffer solution (pH 7 to pH 9). However, another buffer solution may be used without any limitation thereto. In this description, the above-described colloidal gold, onto which an antibody against an analyte or a control antibody (or antigen) is immobilized, is called a "conjugate".

### (Blocking)

The conjugate of the present invention may be blocked by a blocking agent in a region not bound with an antibody of a colloidal gold surface.

The blocking agent of the colloidal gold conjugate is generally a component derived from a living organism or a component derived from a non-living organism, and the component derived from a living organism may be any component as long as it is derived from the living organism and has a blocking effect, and for example, the component includes an animal protein or an animal protein-derived peptide. Specifically, the component may include bovine serum albumin (BSA), Blocking Peptide Fragment (manufactured by TOYOBO) derived from microorganisms, NEO PROTEIN SAVER (manufactured by TOYOBO) derived from silk protein (hydrolysate of sericin), StartingBlock™ (PBS) Blocking Buffer (manufactured by PIERCE), StabilCoat™ (manufactured by SurModics), and casein.

A concentration of the component derived from the living organism may be appropriately determined depending on the component to be used. For example, an antibody solution is added to and mixed with a colloidal gold solution adjusted to 1 OD/mL, and then the component derived from the living organism is added to the mixed solution at a final concentration within a range of 0.1% to 10% for blocking, and more preferably used within a range of 0.2% to 5%.

Alternatively, a mixture of both a component derived from a non-living organism and a component derived from a living organism may be used as a blocking agent of colloidal gold.

The term "detection" or "measurement", as used herein, must be construed in the broadest sense including verification and/or quantification of the presence of the analyte and must not be construed in a limited manner in any sense.

### (Specimen Extraction Solution)

In the present invention, a specimen extraction solution may be used in need of extracting the sample according to the concentration of the analyte in the sample. The specimen extraction solution may be an extraction solution of any composition, as long as it does not significantly inhibit antigen-antibody reaction, or conversely, does not significantly facilitate the reaction resulting in excessive aggregation of the labeling substance, which causes failure of spreading by capillarity, or does not completely prevent antigen concentration-dependent signal detection of the antigen-antibody reaction.

The specimen extraction solution having these actions may be, for example, purified water, physiological saline, or low-concentration buffer solutions such as 10 mmol/L to 20 mmol/L of a phosphate buffer solution, 10 mmol/L to 20 mmol/L of a Tris-HCl buffer solution, or 10 mmol/L to 20 mmol/L of a Bis-Tris buffer solution. Further, a surfactant may be added to the extraction solution for the purpose of controlling a spreading rate of the sample in the strip.

The specimen extraction solution of the present invention may also include a buffer solution having an ability to suppress cross-reactions other than a reaction between the analyte and the antibody specific thereto, as described above. In this case, the specific buffer solution of the present invention may be used as the specimen extraction solution in place of the buffer solution described above.

### (Spreading Solution)

In the present invention, the spreading solution refers to a mixed solution of the specimen solution and the conjugate pad component which spread on the immunochromatographic test strip. When the test strip has a sample pad, the spreading solution may include a sample pad component and may further include an antibody-immobilized membrane component.

Further, the spreading solution of the present invention may include the above-described specific buffer solution of the present invention. In this case, pH and concentration thereof may be in the same range as those of the specific buffer solution. That is, the buffer solution may have pH of 5.0 to 6.7, preferably pH of 5.0 to 6.5, more preferably pH of 5.5 to 6.5, and much more preferably pH of 5.5 to 6.0. The concentration of the buffer solution in the spreading solution is 10 mmol/L or more, and preferably 20 mmol/L to 80 mmol/L.

### (Sample Pad)

In the present invention, a "sample pad" is a part that serves as a sample-supplying portion receiving a sample, and is shaped into a pad to absorb a liquid sample, and the sample pad may include any material and form allowing the passage of a liquid and the analyte component.

In this case, it may be contained at least in a part or entirety of the sample pad.

The sample pad of the present invention may contain the buffer solution of the present invention as described above. In this case, the buffer solution may be contained at least in a part or entirety of the sample pad.

Specific examples of materials suitable for the sample pad may include, but are not limited to, a glass fiber, an acrylic fiber, a hydrophilic polyethylene material, a dry paper, a paper pulp, a fabric, etc. A glass fiber pad is preferably used. The sample pad may additionally be given a function of a conjugate pad described later. The sample pad may contain a commonly used blocking reagent as needed within a range not affecting the reaction system and not departing from the object of the present invention.

### (Conjugate Pad)

The "conjugate pad", as used herein, refers to a pad which is obtained by impregnating a material suitable for the conjugate pad described later with a detection reagent specifically reactive with the analyte, followed by drying. The conjugate pad has a function of allowing the detection reagent and the analyte to form a complex when the sample passes through the conjugate pad. The conjugate pad may by itself be disposed in contact with an antibody-immobilized membrane. Alternatively, the conjugate pad may be disposed in contact with the sample pad so as to receive the sample which has passed through the sample pad by a capillary flow and then transfer the sample by a capillary flow to another pad (hereinafter, also referred to as a "third pad") in contact with the surface different from the contact surface with the sample pad. The selection of one or more parts of the sample pad and the conjugate pad and how the selected parts are disposed on the antibody-immobilized membrane may be appropriately changed.

The conjugate pad of the present invention may also contain the buffer solution as described above. In this case, the buffer solution is contained at least in a part including the upstream side from a part to which the conjugate is immobilized, and may be contained in entirety of the conjugated pad.

Materials suitable for the conjugate pad may include, but are not limited to, paper, a cellulose mixture, nitrocellulose, polyester, an acrylonitrile copolymer, a glass fiber, and a nonwoven fiber such as rayon. A glass fiber pad is preferably used.

The conjugate pad may contain, as needed, a "control reagent" for ensuring reliability of immunochromatography, for example, an antibody labeled with a label and not reactive with the sample component, or a highly antigenic protein such as KLH (keyhole limpet hemocyanin) labeled with a label. These control reagents are components (substances) having no possibility of being present in the sample and may appropriately be selected.

### (Third Pad)

In the present invention, a third pad may be disposed for the purpose of removing components unnecessary for detection of the analyte from the reaction components of the sample and the detection reagent so that components necessary for reaction may smoothly spread in an insoluble membrane to which an antibody is immobilized.

For example, blood cells, insoluble blood cell fractures, etc. are preferably removed as the components unnecessary for detection. The third pad may also be given an additional effect of preliminarily removing aggregates, which are grown to a size which prevents the movement to and the smooth spread in the antibody-immobilized membrane, out of aggregates generated by antigen-antibody reactions. The third pad may include any material and form allowing the passage of the components of the liquid and the analyte component.

Specific examples include, but are not limited to, a glass fiber, an acrylic fiber, a hydrophilic polyethylene material, a dry paper, a paper pulp, a fabric, etc.

The third pad of the present invention may contain the buffer solution as described above. In this case, the buffer solution may be contained at least in a part or entirety of the third pad.

### (Immobilization of Antibody to Insoluble Membrane)

In the immunochromatographic reagent of the present invention, immobilization of the antibody against the analyte to the insoluble membrane may be performed by a generally well-known method. For example, in the case of the flow-through format, the antibody is prepared at a predetermined concentration and a given amount of the solution thereof is applied to the insoluble membrane in a shape of dot or a specific symbol such as "+". In this case, to ensure reliability of immunochromatography, a "control line" is generally formed by immobilizing a protein or a compound capable of binding to the conjugate to a position different from the antibody against the analyte. The "control line" may be formed by immobilizing the antibody against the control reagent to a position different from the antibody against the analyte.

In the case of a lateral-flow format, the antibody is prepared at a predetermined concentration and the solution thereof is applied to an insoluble membrane in a line shape by using a device having a mechanism capable of horizontally moving while discharging the solution from a nozzle at a constant rate. In this regard, a concentration of the antibody is preferably 0.1 mg/mL to 5 mg/mL, and more preferably 0.5 mg/mL to 3 mg/mL. An immobilized amount of the antibody on the insoluble membrane may be optimized by adjusting the application amount dropped onto the insoluble membrane in the case of the flow-through format, and optimized by adjusting the discharge rate from the nozzle of the device in the case of the lateral-flow format. Particularly, in the case of the lateral-flow format, 0.5 µL/cm to 2 µL/cm is preferable. In the present invention, a "flow-through membrane assay" refers to a format in which the sample liquid, etc. spreads to perpendicularly pass through the insoluble membrane, and a "lateral-flow membrane assay" refers to a format in which the sample liquid, etc. spreads to move in parallel with the insoluble membrane.

In the present invention, the application position of an antibody against a target object to the insoluble membrane may be placed such that the detection reagent spreads from the conjugate pad by capillarity and sequentially passes through the lines to which the respective antibodies are applied in the case of the lateral-flow format. Preferably, the line formed by applying the antibody against the analyte is preferably located upstream while the line formed by applying a control antibody is located downstream thereof. In this case, a sufficient distance is preferably placed between the respective lines such that signals of labels may be detected. In the case of the flow-through format, the position of application of the antibody against the target object may be placed such that signals of labels may be detected.

The antibody solution applied to the insoluble membrane may normally be prepared by using a predetermined buffer solution. A type of the buffer solution may include commonly used buffer solutions such as a phosphate buffer solution, a Tris buffer solution, a Good's buffer solution, etc. pH of the buffer solution is preferably in a range of 6.0 to 11 and may be appropriately determined depending on properties of the antibody to be used. For example, a buffer solution of pH 7.2 is usable for an anti-fFN monoclonal antibody described later.

The buffer solution may contain a salt such as NaCl, etc. , a stabilizer such as sucrose or a preservative, and antiseptic such as ProClin, etc. The salt may include those contained for adjusting ionic strength, such as NaCl, as well as those added at the step of adjusting pH of the buffer solution, such as sodium hydroxide. After the antibody is immobilized to the insoluble membrane, blocking may be performed by coating a portion other than the antibody-immobilized parts with a commonly used blocking agent in a solution or in a vapor state. In this description, the insoluble membrane having the antibody immobilized as described above is also referred to as an "antibody-immobilized membrane".

### (Insoluble Membrane)

In the present invention, the insoluble membrane (hereinafter, also simply referred to as a membrane) may be made of any material. For example, the materials may include, but are not limited to, polyethylene, polyethylene terephthalate, nylons, glass, polysaccharide such as cellulose and cellulose derivatives, or ceramics. Specifically, the materials may include glass fiber filter papers and cellulose filter papers available from Merck & Co. , Inc., Toyo Roshi kaisha, Ltd., Whatman, Inc., etc. A pore size and structure of the insoluble membrane may be appropriately selected, thereby controlling a flow speed of an immune complex of a colloidal gold-labeled antibody and a target object through the membrane. The amount of the labeled antibody binding to the antibody immobilized to the membrane may be adjusted by controlling the flow speed through the membrane, and therefore, the pore size and the structure of the membrane are preferably optimized in consideration of combinations with the other constituent materials of the immunochromatographic test strip of the present invention.

Further, as described above, the insoluble membrane of the present invention may include the buffer solution. In this case, the buffer solution may be contained at least in a part of the upstream side from a detection part, to which the antibody is immobilized, in the insoluble membrane, and may be contained in entirety of the insoluble membrane.

### (Absorption Pad)

In the present invention, the absorption pad refers to a liquid-absorbing part that absorbs the sample which has moved on and passed through the insoluble membrane to control the spread of the sample. The absorption pad may be disposed at the most downstream portion of the strip configuration in the lateral-flow format, and may be disposed on, for example, the lower portion of the antibody-immobilized membrane in the flow-through format. The absorption pad may be made of, for example, filter paper, but is not limited thereto.

### (Detection Device)

The immunochromatographic test strip of the present invention may be used after being housed/mounted in an appropriate container (housing) in consideration of a size of the strip, an addition method and position of the sample, an immobilization position of antibody on the antibody-immobilized membrane, a signal detection method, etc., and the immunochromatographic test strip in such a housed/mounted state is referred to as a "device".

### (Others)

In this description, the "insoluble membrane" is also referred to as a "solid phase", and allowing the insoluble membrane to physically or chemically support antigens or antibodies or a state of allowing the support may be expressed as "immobilization", "immobilized", "solid-phased", "sensitization", or "adsorption".

### (Antibody Used in the Present Invention)

The antibody against the analyte used in the present invention is not limited in any way to a preparation method as long as the antibody is specifically reactive to the analyte, and may be a polyclonal antibody or a monoclonal antibody. A hybridoma producing the corresponding antibody may be generally prepared by cell fusion between spleen cells of an animal immunized with the analyte as an immunogen and myeloma cells from the same species in accordance with the method of Kohler and Milstein (see Nature, Vol. 256, p. 495, 1975).

When antibodies used in a measurement method of detecting the analyte through formation of so-called sandwich are monoclonal antibodies, a relationship between an antibody labeled with a labeling substance (first antibody) included in the conjugate and an insoluble membrane-immobilized antibody (second antibody) to be immobilized in the spreading portion is such that the epitope of the second antibody is different from that of the first antibody. It is preferable that any one of the first and second antibodies is an anti-analyte antibody, and the other is an antibody binding to the analyte. It is more preferable that the first antibody is the antibody binding to the analyte, and the second antibody is the anti-analyte antibody. The antibody binding to the analyte may be any antibody binding to the analyte, and any antibody binding to an antigen similar to the analyte, with which the sample may be contaminated.

In Example 1 described later, fFN is supposed as an analyte protein, and plasma FN is supposed as an antigen similar thereto. An anti-fFN monoclonal antibody that specifically binds to fFN and does not bind to plasma FN (hereinafter, referred to as an anti-fFN monoclonal antibody) and an anti-fibronectin antibody that binds to both fFN and plasma FN (hereinafter, referred to as an anti-FN antibody) are used.

fFN and plasma FN are glycoproteins that have the same amino acid sequence, but differ only in the sugar chain content. Here, in the collection of a vaginal secretion, etc., which is a specimen containing fFN, of a pregnant woman, there is a possibility of blood contamination, and therefore, the specimen contains both plasma FN and fFN. It is difficult to obtain two or more kinds of the fFN-specific antibodies because such antibodies recognize the difference of the sugar chain. Therefore, when the antibodies are used in a sandwich immunochromatography, it may be necessary to construct an assay system with two kinds of antibodies, in which one is an anti-oncofetal FN monoclonal antibody and the other is an anti-FN monoclonal antibody.

The anti-fFN monoclonal antibody used in the present invention is a monoclonal antibody, which is obtained by synthesizing a glycopeptide containing a sugar chain binding site peculiar to fFN, binding it to transferrin or OVA by a carbodiimide method, and performing footpad immunization and subcutaneous immunization with the resulting product as an immunogen, and specifically reacts with fFN. Further, the anti-FN monoclonal antibody used in the present invention is a monoclonal antibody, which is obtained by similarly performing immunization with a full-length protein of plasma FN as an immunogen, and reacts with any one of oncofetal FN and plasma FN.

The present invention is not limited thereto, and a commercially available anti-FN monoclonal antibody and anti-fFN monoclonal antibody may also be used. Examples of the commercially available anti-FN monoclonal antibody may include Anti-Human Fibronectin, Monoclonal (Clone FN12-8), Anti-Human Fibronectin, Monoclonal (Clone FN30-8) (manufactured by Takara Bio, Inc.) (It is noted that monoclonal antibodies may be denoted by clone names of hybridomas producing the respective antibodies for convenience. The same applies hereinafter).

When oncofetal fibronectin is detected, any one of the first antibody and the second antibody is preferably the anti-fFN monoclonal antibody, and the other is preferably the anti-FN monoclonal antibody, and more preferably, the first antibody is the anti-FN monoclonal antibody, and the second antibody is the anti-fFN monoclonal antibody.

### (Preparation Example of Anti-fFN Monoclonal Antibody and Anti-FN Monoclonal Antibody)

The anti-fFN monoclonal antibody (Clone # 90204) used in the following test was obtained by immunizing mice with fFN peptide fragment-bound OVA or fFN peptide fragment-bound transferrin, and the anti-FN monoclonal antibody (Clone # 90412) was obtained by immunizing mice with a full-length protein of plasma fibronectin according to a method which is generally performed by those skilled in the art in order to prepare monoclonal antibodies (method of Kohler and Milstein, (Nature, Vol. 256, p. 495, 1975)).

### (Measurement)

A method of quantifying signals derived from colloidal gold may be performed in accordance with a known method, and absorbance or reflected light intensity may be measured. Alternatively, the changes in absorbance or reflected light intensity may be extrapolated to a calibration curve of samples with known concentrations to measure the concentration of the target object.

### (Kit)

A combination of the immunochromatographic test strip, and any one or more of the specimen extraction solution, and an instruction manual may be used as a kit. In this case, the buffer solution component of the present invention may be contained in any one or more of the conjugate pad, the sample pad, and the specimen extraction solution of the immunochromatographic test strip.

### (Detection Method Using Immunochromatography)

The detection method using immunochromatography of the present invention is a method comprising at least the following steps of (A) to (C), and hereinbelow, a typical detection method using fFN as an analyte and plasma FN as a similar antigen will be described:
(A) supplying a sample to a sample-supplying portion of a test strip comprising a membrane consisting of a porous body equipped with at least the sample-supplying portion, a spreading portion, and a detecting portion, wherein a colloidal gold-labeled anti-FN antibody (conjugate) is retained in a part of the spreading portion in a dissoluble manner, and the detecting portion in which an anti-fFN antibody is immobilized is provided in a part of the spreading portion on the downstream side relative to the conjugate-retaining part;
(B) bringing the sample (specimen) into contact with the conjugate in the presence of a buffer solution having an ability to suppress cross-reactions between plasma FN in the sample and anti-fFN antibody; and
(C) detecting a complex of fFN in the sample component and the colloidal gold-labeled anti-FN antibody (conjugate) obtained in step (B) in the detecting portion to which the anti-fFN antibody is immobilized.

### [EXAMPLE]

### [Test Example 1] Production of Immunochromatographic Test Strip of the Present Invention

### 1. Preparation of Colloidal Gold-Labeled Anti-FN Antibody Solution

The preparation was performed by the following procedure.
(i) A 50-nm colloidal gold stock solution was diluted with purified water to 1.0 OD, and adjusted to pH 8.5 with a 0.2 M potassium carbonate aqueous solution.
(ii) Anti-plasma FN mouse monoclonal antibody (clone # 90412) was diluted with 5 mM boric acid to be 80 µg/mL.
(iii) BSA was dissolved in purified water so as to be a 10% solution.
(iv) 1 mL of (ii) was added to 20 mL of (i) under stirring, followed by stirring for 10 minutes.
(v) 2 mL of (iii) was added to (iv) under stirring, followed by stirring for 5 minutes.
(vi) (v) was centrifuged at 10°C and 10,000 rpm for 45 minutes, the supernatant was aspirated, and the precipitate was suspended with 1 mL of a conjugate-diluting solution (Buffer Conjugate Dilution, SCRIPPS) to obtain a colloidal gold-labeled anti-FN antibody solution (Anti-FN antibody conjugate).
(vii) (vi) was heated at 45°C for 18 hours.

### 2. Production of Conjugate Pad

The colloidal gold-labeled anti-FN antibody solution prepared in 1 was mixed with a 20 mM aqueous solution of monosodium phosphate containing 2.0% BSA and a 2.4% lactose solution so as to be 3.75 OD/mL, to prepare a conjugate solution (pH 7). The conjugate solution was impregnated into a glass fiber pad on a cookie sheet at a rate of 66.93 µL/cm², and dried in a dry oven at 70°C for 45 minutes, and this pad was used as a conjugate pad.

### 3. Production of Sample Pad

A 20 mM aqueous solution of monosodium phosphate (pH 7) was impregnated into a glass fiber pad on a cookie sheet at a rate of 62.99 µL/cm², and dried in a dry oven at 70°C for 45 minutes, and this pad was used as a sample pad.

### 4. Preparation of Anti-fFN Antibody and Anti-mouse IgG Antibody Membrane Application Solutions

### (i) Preparation of anti-fFN antibody membrane application solution

An anti-fFN mouse monoclonal antibody (clone # 90204) was diluted with a 10 mM phosphate buffer (pH 7.2) containing 2.5% sucrose so that the antibody concentration became 2.0 mg/mL, and this solution was used as an anti-fFN antibody membrane application solution.

### (ii) Preparation of anti-mouse IgG antibody membrane application solution

As in (i), an anti-mouse IgG goat polyclonal antibody (Jackson Immuno Research Laboratories) was diluted with a 10 mM phosphate buffer (pH 7.2) containing 2.5% sucrose so that the antibody concentration became 0.5 mg/mL, and this solution was used as an anti-mouse IgG goat polyclonal antibody membrane application solution.

### 5. Production of Antibody-Immobilized Membrane

An immunochromatography dispenser XYZ3000 (BIO DOT) was used to apply the anti-fFN antibody membrane application solution and the anti-mouse IgG antibody membrane application solution at a position of 11 mm and 15 mm distant from the lower end of a nitrocellulose membrane (Hi-Flow Plus HF180 (Merck Millipore)) at 1.0 µL/cm, respectively and dried in a dry oven at 70°C for 45 minutes to produce an antibody-immobilized membrane.

### 6. Production of Test Device

To a plastic adhesive sheet (g), the antibody-immobilized membrane (d) was affixed, antibody-application sites were arranged in the order of the anti-fFN antibody (e) on the upstream side of spread, and then the anti-mouse IgG antibody (j) as a control reagent, and a third pad (c) was mounted. Next, the above-prepared conjugate pad (b) was placed and mounted, and the above-prepared sample pad (a) was placed and mounted so as to overlap with this conjugate pad, while the absorption pad (f) was placed and mounted on the end of the other side. A transparent plastic film (h) was placed on the surface of the antibody-immobilized membrane and the absorption pad, and an immunochromatographic test strip was produced by cutting a structure having the constituting elements overlapped with each other as described above.

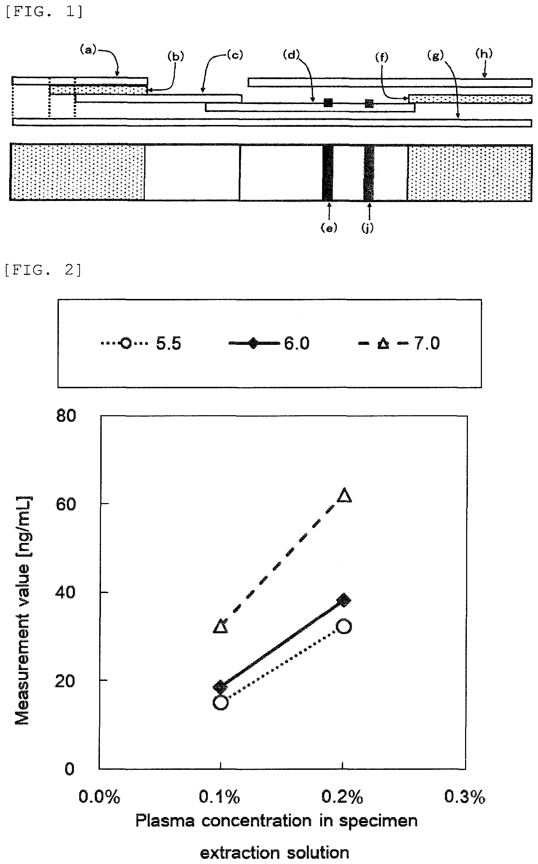

The test strip was housed/mounted in a dedicated plastic housing having a sample-addition window and a detection window (DKVIN (Syn corporation) (not shown in FIG. 1)) at the time of an assay to implement a form of an immunochromatographic test device. FIG. 1 shows a schematic structure of the immunochromatographic test strip.

### [Example 1] Study of pH of Conjugate Pad Application Solution

pH of the buffer of the conjugate pad application solution was changed to examine the effect on sensitivity and cross-reaction with plasma FN.

### 1. Test Method

### (1) Production of Test Device

A test device was produced in the same manner, except that the final concentration of the aqueous solution of monosodium phosphate in "Production of Immunochromatographic Test Device" of the present invention, "2. Production of Conjugate Pad", and "3. Production of Sample Pad" was adjusted to "20 mM phosphate (pH 5.5)", "20 mM phosphate (pH 6.0)", "20 mM phosphate (pH 7.0)", or "20 mM phosphate (pH 8.0)".

### (2) Preparation of Specimen

### (2-1) Test for measuring fFN detection sensitivity

With respect to the following commercially available human amniotic fluid, the fFN concentration was calculated with an immunotester, and the human amniotic fluid was added to the fFN specimen extraction solution to be 50 ng/mL, which was used as a specimen.

Material;
Commercially available human amniotic fluid (Golden West Biologicals, Inc.)
fFN specimen extraction solution (specimen extraction solution for immunotester)
Evaluation criteria for measurement sensitivity are as follows.

### Symbols Meaning of symbols

"-" Undetectable
"+" detectable
"++" detectable and high detection sensitivity

### (2-2) Test for measuring cross-reaction with plasma FN

A plasma pool of healthy people was diluted with the fFN specimen extraction solution to prepare specimens of 0.1% and 0.2% plasma.

### (3) Measurement

120 µL of each specimen was dropped to the test device, and after 10 minutes, the reflection absorbance of the test line and the control line was measured with RAPIDPIA ((registered trademark) Sekisui Medical Co., Ltd.) and software (C10066).

### 2. Test Results

Results are shown in FIG. 2 and Table 1.

In the test device where the pH of phosphate of the conjugate pad application solution was lowered to 6.0, cross-reactivity with plasma FN was decreased while maintaining detection sensitivity for fFN, as compared with the device of pH 7.0.

Further, in the test device where the pH was lowered to 5.5, cross-reactivity with plasma FN was decreased, as compared with the device of pH 6.0.

**[Table 1]**

| Buffer | | Sensitivity |
|---|---|---|
| 20 mM phosphate | pH 7.0 | ++ |
| | pH 6.0 | ++ |
| | pH 5.5 | ++ |

### [Example 2] Study of Buffer Concentration at Specific pH

The pH of the buffer of the conjugate pad application solution was fixed and the concentration of the buffer was changed to examine the effect on cross-reactivity with plasma FN.

### 1. Test Method

### (1) Production of Test Device

A test device was produced in the same manner, except that the final concentration of the aqueous solution of monosodium phosphate in "Production of Immunochromatographic Test Device" of the present invention, "2. Production of Conjugate Pad", and "3. Production of Sample Pad" was adjusted to "20 mM phosphate (pH 6.0)", "40 mM phosphate (pH 6.0)", or "80 mM phosphate (pH 6.0)". A test device of "20 mM phosphate (pH 7.0)" was used for reference.

### (2) Preparation of Specimen

In the same manner as in Example 1 (2-2).

### (3) Measurement

In the same manner as in Example 1.

### 2. Test Results

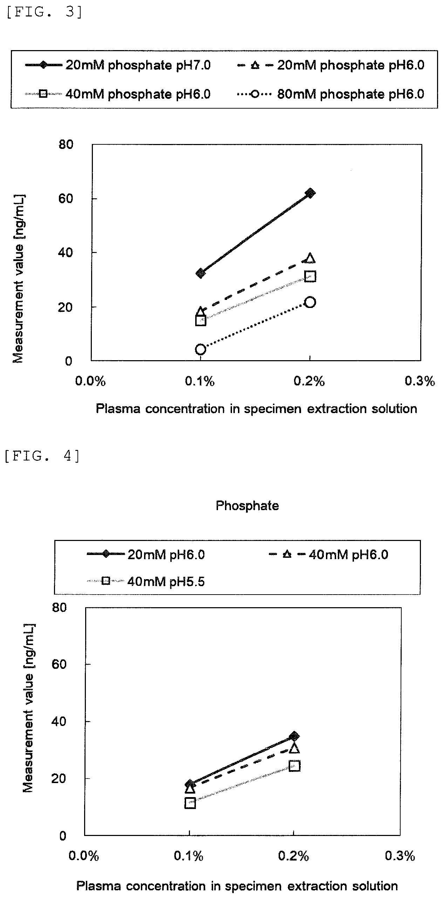

Results are shown in Table 2 and FIG. 3.

It was found that when phosphate (pH 6.0) was used as the conjugate pad application solution, cross-reactivity with plasma FN was reduced while maintaining detection sensitivity for fFN at a detectable level by increasing the concentration of the buffer.

**[Table 2]**

| Buffer | | Sensitivity |
|---|---|---|
| Phosphate pH 7.0 (For reference) | 20mM | ++ |
| Phosphate pH 6.0 | 20mM | ++ |
| | 40mM | ++ |
| | 80mM | + |

### [Example 3] Study of Kind, Concentration, and pH of Buffer

The kind, concentration, and pH of the buffer of the conjugate pad application solution were changed to examine the effects on the sensitivity and cross-reactivity with plasma FN.

### 1. Test Method

### (1) Production of Test Device

A test device was produced in the same manner, except that the final concentration of the aqueous solution of monosodium phosphate in "Production of Immunochromatographic Test Device" of the present invention, "2. Production of Conjugate Pad", and "3. Production of Sample Pad" was adjusted to "20 mM phosphate (pH 6.0)" "40 mM phosphate (pH 6.0)", "40 mM phosphate (pH 5.5)", "20 mM ADA (pH 6.0)", "40 mM ADA (pH 6.0)", "40 mM ADA (pH 5.5)", "20 mM MES (pH 6.0)", "40 mM MES (pH 6.0)", or "40 mM MES (pH 5.5)".

### (2) Preparation of Specimen

In the same manner as in Example 1.

### (3) Measurement

In the same manner as in Example 1.

### 2. Test Results

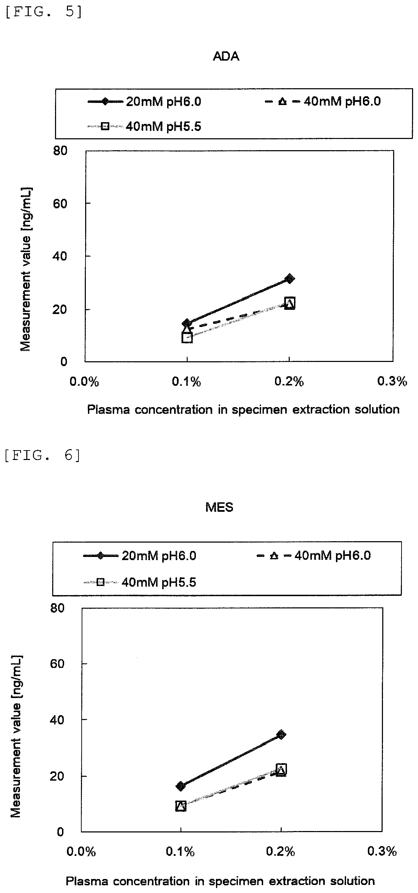

Results are shown in Table 3 and FIGS. 4, 5, and 6.

When phosphate was used as the conjugate pad application solution, detection sensitivity for fFN at 40 mM (pH 6.0) or 40 mM (pH 5.5) was the same as that at 20 mM (pH 6.0), and cross-reactivity with plasma FN was similarly low.

When ADA was used, detection sensitivity for fFN at 20 mM (pH 6.0), 40 mM (pH 6.0), or 40 mM (pH 5.5) was the same as that of phosphate (pH 6.0), and cross-reactivity with plasma FN was also low similarly to phosphate.

When MES was used, detection sensitivity for fFN at 20 mM (pH 6.0) or 40 mM (pH 6.0) was the same as that of phosphate (pH 6.0). Further, when MES at 40 mM (pH 5.5) was used, detectable fFN sensitivity was obtained, and cross-reactivity with plasma FN was also low.

Accordingly, it was found that cross-reactivity with plasma FN was suppressed while maintaining detection sensitivity for fFN by decreasing pH or increasing the buffer concentration of ADA, MES, or phosphate.

**[Table 3]**

| Buffer | | | Sensitivity |
|---|---|---|---|
| Type | Concentration | pH | |
| Phosphate | 20mM | 6.0 | ++ |
| | 40mM | 6.0 | ++ |
| | 4 0mM | 5.5 | ++ |
| ADA | 20mM | 6.0 | ++ |
| | 40mM | 6.0 | ++ |
| | 40mM | 5.5 | ++ |
| MES | 20mM | 6.0 | ++ |
| | 40mM | 6.0 | ++ |
| | 40mM | 5.5 | + |

### INDUSTRIAL APPLICABILITY

In a detection method using immunochromatography, even when similar antigens are present, cross-reactions between an antibody against an analyte and the similar antigens may be reduced and measurement value errors and false positive reactions may be prevented, while the detection sensitivity for the analyte may be maintained or improved and accurate determinations may be allowed.

Particularly, in a method of detecting oncofetal fibronectin (fFN) using immunochromatography, even when a specimen is contaminated with blood, measurement errors and false positive reactions hardly occur and the detection sensitivity for fFN may be maintained or improved and accurate determinations may be allowed. Accordingly, it is possible to provide a detection reagent for threatened premature labor using immunochromatography which is frequently used in POCT.

### REFERENCE NUMERALS

(a) Sample pad
(b) Conjugate pad
(c) Third pad
(d) Antibody-immobilized membrane
(e) anti-fFN antibody
(f) Absorption pad
(g) Plastic adhesive sheet
(h) Plastic film
(j) Control antibody

## Claims

1. A detection method using immunochromatography, comprising steps (A) to (C):
(A) supplying a sample possibly containing an analyte and a substance similar to the analyte to a sample-supplying portion of the following test strip;
the test strip comprising a membrane consisting of a porous body equipped with at least the sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, wherein any one of the first antibody and the second antibody is an anti-analyte antibody and the other is an antibody binding to the analyte, and wherein the antibody binding to the analyte may be any antibody binding to the analyte and any antibody binding to an antigen similar to the analyte;
(B) bringing the analyte in the sample into contact with the conjugate in the presence of a buffer solution of pH 5.0 to pH 6.7; and
(C) detecting a complex of the analyte in the sample and the conjugate in the detecting portion;
wherein the analyte is oncofetal fibronectin, and the substance similar to the analyte is plasma fibronectin.

2. The detection method of claim 1, wherein the first antibody is an anti-fibronectin monoclonal antibody, and the second antibody is an anti-oncofetal fibronectin monoclonal antibody.

3. The detection method of any one of claims 1 to 2, wherein the buffer solution is any one or more selected from the group consisting of phosphate, ADA, MES, ACES, Bis-Tris, citrate, HEPES, MOPS, PIPES, and Tris buffer solution.

4. An immunochromatographic test strip comprising the following components (1) and (2):
(1) a membrane consisting of a porous body equipped with at least a sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, wherein any one of the first antibody and the second antibody is an anti-analyte antibody and the other is an antibody binding to the analyte, and wherein the antibody binding to the analyte may be any antibody binding to the analyte and any antibody binding to an antigen similar to the analyte; and
(2) a buffer solution component of pH 5.0 to pH 6.7 which is contained in at least a part of a portion from the sample-supplying portion to the detecting portion of the spreading portion;
wherein the analyte is oncofetal fibronectin and the substance similar to the analyte is plasma fibronectin.

5. The test strip of claim 4, wherein a sample pad, a conjugate pad, and an antibody-immobilized membrane are arranged in this order from the upstream, the sample pad includes the sample-supplying portion, the conjugate pad includes the conjugate-retaining part and the buffer solution component, and the antibody-immobilized membrane includes the detecting portion.

6. The test strip of claim 4 or 5, wherein any one of the first antibody and the second antibody is an anti-fibronectin monoclonal antibody, and the other is an anti-oncofetal fibronectin monoclonal antibody.

7. The test strip of claim 6, wherein the first antibody is an anti-fibronectin monoclonal antibody, and the second antibody is an anti-oncofetal fibronectin monoclonal antibody.

8. The test strip of any one of claims 5 to 7, wherein the buffer solution is any one or more selected from the group consisting of phosphate, ADA, MES, ACES, Bis-Tris, citrate, HEPES, MOPS, PIPES, and Tris buffer solution.

9. An immunochromatographic detection kit comprising the following components (1) and (2):
(1) an immunochromatographic test strip comprising a membrane consisting of a porous body equipped with at least a sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, wherein any one of the first antibody and the second antibody is an anti-analyte antibody and the other is an antibody binding to the analyte, and wherein the antibody binding to the analyte may be any antibody binding to the analyte and any antibody binding to an antigen similar to the analyte; and
(2) a buffer solution of pH 5.0 to pH 6.7;
wherein the analyte is oncofetal fibronectin, the substance similar to the analyte is plasma fibronectin.

10. The detection kit of claim 9, wherein any one of the first antibody and the second antibody is an anti-fibronectin monoclonal antibody, and the other is an anti-oncofetal fibronectin monoclonal antibody.

11. The detection kit of claim 9 or 10, wherein the buffer solution is used as a spreading solution or a specimen extraction solution.

12. The detection kit of any one of claims 9 to 11, wherein the buffer solution is any one or more selected from the group consisting of phosphate, ADA, MES, ACES, Bis-Tris, citrate, HEPES, MOPS, PIPES, and Tris buffer solution.

13. A method of reducing cross-reaction between an analyte and a substance similar to the analyte, in a method of detecting an analyte in a sample using an immunochromatographic test strip,
the method of reducing cross-reaction comprising the following steps:
(A) supplying a sample possibly containing the analyte and the substance similar to the analyte to a sample-supplying portion of the following test strip;
the test strip comprising a membrane consisting of a porous body equipped with at least a sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, wherein any one of the first antibody and the second antibody is an anti-analyte antibody and the other is an antibody binding to the analyte, and wherein the antibody binding to the analyte may be any antibody binding to the analyte and any antibody binding to an antigen similar to the analyte;
(B) bringing the analyte in the sample into contact with the conjugate in the presence of a buffer solution of pH 5.0 to pH 6.7; and
(C) detecting a complex of the analyte in the sample and the conjugate in the detecting portion;
wherein the analyte is oncofetal fibronectin, and the substance similar to the analyte is plasma fibronectin.

14. The method of claim 13, wherein the first antibody is an anti-fibronectin monoclonal antibody, and the second antibody is an anti-oncofetal fibronectin monoclonal antibody.

15. The method of any one of claims 13 to 14, wherein the buffer solution is any one or more selected from the group consisting of phosphate, ADA, MES, ACES, Bis-Tris, citrate, HEPES, MOPS, PIPES, and Tris buffer solution.

16. A spreading solution for immunochromatography for detecting oncofetal fibronectin, the spreading solution comprising the following (1) and (2) :
(1) a buffer solution of pH 5.0 to pH 6.7, and
(2) a conjugate containing a first antibody labeled with a labeling substance.

17. Use of a specimen extraction solution comprising a buffer solution of pH 5.0 to pH 6.7 for detecting oncofetal fibronectin.

## Patentansprüche

1. Detektionsverfahren unter Verwendung von Immunchromatographie, umfassend die Schritte (A) bis (C) :
(A) Zuführen einer Probe, die möglicherweise einen Analyten und eine dem Analyten ähnliche Substanz enthält, zu einem Proben-zuführenden Abschnitt des folgenden Teststreifens;
wobei der Teststreifen eine Membran umfasst, bestehend aus einem porösen Körper, ausgestattet mit wenigstens dem Proben-zuführenden Abschnitt, einem Verteilungsabschnitt und einem Detektionsabschnitt, wobei ein Konjugat, welches einen ersten Antikörper enthält, der mit einer Markierungssubstanz markiert ist, in einem Teil des Verteilungsabschnitts in lösbarer Weise gehalten wird, und der Detektionsabschnitt, in welchem ein zweiter Antikörper immobilisiert ist, in einem Teil des Verteilungsabschnitts auf der abstromigen Seite relativ zu dem das Konjugat haltenden Teil bereitgestellt ist, wobei irgendeiner von dem ersten Antikörper und dem zweiten Antikörper ein Anti-Analyten-Antikörper ist und der andere ein an den Analyten bindender Antikörper ist, und wobei der an den Analyten bindende Antikörper irgendein an den Analyten bindender Antikörper und irgendein an ein dem Analyten ähnliches Antigen bindender Antikörper sein kann;
(B) Inkontaktbringen des Analyten in der Probe mit dem Konjugat in der Gegenwart einer Pufferlösung mit einem pH-Wert von 5,0 bis 6,7; und
(C) Detektieren eines Komplexes des Analyten in der Probe und des Konjugats in dem Detektionsabschnitt;
wobei der Analyt onkofetales Fibronektin ist und die dem Analyten ähnliche Substanz Plasmafibronektin ist.

2. Detektionsverfahren nach Anspruch 1, wobei der erste Antikörper ein monoklonaler Anti-Fibronektin-Antikörper ist und der zweite Antikörper ein monoklonaler Antionkofetales-Fibronektin-Antikörper ist.

3. Detektionsverfahren nach irgendeinem der Ansprüche 1 bis 2, wobei die Pufferlösung irgendeine oder mehrere ist, ausgewählt aus der Gruppe, bestehend aus Phosphat, ADA, MES, ACES, Bis-Tris, Citrat, HEPES, MOPS, PIPES und Tris-Pufferlösung.

4. Immunchromatographischer Teststreifen, umfassend die folgenden Komponenten (1) und (2):
(1) eine Membran, bestehend aus einem porösen Körper, ausgestattet mit wenigstens dem Proben-zuführenden Abschnitt, einem Verteilungsabschnitt und einem Detektionsabschnitt, wobei ein Konjugat, welches einen ersten Antikörper enthält, der mit einer Markierungssubstanz markiert ist, in einem Teil des Verteilungsabschnitts in lösbarer Weise gehalten wird, und der Detektionsabschnitt, in welchem ein zweiter Antikörper immobilisiert ist, in einem Teil des Verteilungsabschnitts auf der abstromigen Seite relativ zu dem das Konjugat haltenden Teil bereitgestellt ist, wobei irgendeiner von dem ersten Antikörper und dem zweiten Antikörper ein Anti-Analyten-Antikörper ist und der andere ein an den Analyten bindender Antikörper ist, und wobei der an den Analyten bindende Antikörper irgendein an den Analyten bindender Antikörper und irgendein an ein dem Analyten ähnliches Antigen bindender Antikörper sein kann; und
(2) eine Pufferlösungskomponente mit einem pH-Wert von 5,0 bis 6,7, die in wenigstens einem Teil eines Abschnitts von dem Proben-zuführenden Abschnitt zu dem Detektionsabschnitt des Verteilungsabschnitts enthalten ist;
wobei der Analyt onkofetales Fibronektin ist und die dem Analyten ähnliche Substanz Plasmafibronektin ist.

5. Teststreifen nach Anspruch 4, wobei ein Probenkissen, ein Konjugatkissen und eine Antikörper-immobilisierte Membran in dieser Reihenfolge von oben her (from the upstream) angeordnet sind, wobei das Probenkissen den Proben-zuführenden Abschnitt einschließt, das Konjugatkissen den das Konjugat haltenden Teil und die Pufferlösungskomponente einschließt und die Antikörper-immobilisierte Membran den Detektionsabschnitt einschließt.

6. Teststreifen nach Anspruch 4 oder 5, wobei irgendeiner von dem ersten Antikörper und dem zweiten Antikörper ein monoklonaler Anti-Fibronektin-Antikörper ist und der andere ein monoklonaler Anti-onkofetales-Fibronektin-Antikörper ist.

7. Teststreifen nach Anspruch 6, wobei der erste Antikörper ein monoklonaler Anti-Fibronektin-Antikörper ist und der zweite Antikörper ein monoklonaler Anti-onkofetales-Fibronektin-Antikörper ist.

8. Teststreifen nach irgendeinem der Ansprüche 5 bis 7, wobei die Pufferlösung irgendeine oder mehrere ist, ausgewählt aus der Gruppe, bestehend aus Phosphat, ADA, MES, ACES, Bis-Tris, Citrat, HEPES, MOPS, PIPES und Tris-Pufferlösung.

9. Immunchromatographisches Detektionskit, umfassend die folgenden Komponenten (1) und (2):
(1) einen immunchromatographischen Teststreifen, umfassend eine Membran, bestehend aus einem porösen Körper, ausgestattet mit wenigstens einem Proben-zuführenden Abschnitt, einem Verteilungsabschnitt und einem Detektionsabschnitt, wobei ein Konjugat, welches einen ersten Antikörper enthält, der mit einer Markierungssubstanz markiert ist, in einem Teil des Verteilungsabschnitts in lösbarer Weise gehalten wird, und der Detektionsabschnitt, in welchem ein zweiter Antikörper immobilisiert ist, in einem Teil des Verteilungsabschnitts auf der abstromigen Seite relativ zu dem das Konjugat haltenden Teil bereitgestellt ist, wobei irgendeiner von dem ersten Antikörper und dem zweiten Antikörper ein Anti-Analyten-Antikörper ist und der andere ein an den Analyten bindender Antikörper ist, und wobei der an den Analyten bindende Antikörper irgendein an den Analyten bindender Antikörper und irgendein an ein dem Analyten ähnliches Antigen bindender Antikörper sein kann; und
(2) eine Pufferlösung mit einem pH-Wert von 5,0 bis 6,7;
wobei der Analyt onkofetales Fibronektin ist und die dem Analyten ähnliche Substanz Plasmafibronektin ist.

10. Detektionskit nach Anspruch 9, wobei irgendeiner von dem ersten Antikörper und dem zweiten Antikörper ein monoklonaler Anti-Fibronektin-Antikörper ist und der andere ein monoklonaler Anti-onkofetales-Fibronektin-Antikörper ist.

11. Detektionskit nach Anspruch 9 oder 10, wobei die Pufferlösung als Verteilungslösung oder Probenextraktionslösung verwendet wird.

12. Detektionskit nach irgendeinem der Ansprüche 9 bis 11, wobei die Pufferlösung irgendeine oder mehrere ist, ausgewählt aus der Gruppe, bestehend aus Phosphat, ADA, MES, ACES, Bis-Tris, Citrat, HEPES, MOPS, PIPES und Tris-Pufferlösung.

13. Verfahren zum Reduzieren von Kreuzreaktion zwischen einem Analyten und einer dem Analyten ähnlichen Substanz in einem Verfahren zur Detektion eines Analyten in einer Probe unter Verwendung eines immunchromatographischen Teststreifens,
wobei das Verfahren zum Reduzieren von Kreuzreaktion die folgenden Schritte umfasst:
(A) Zuführen einer Probe, die möglicherweise den Analyten und die dem Analyten ähnliche Substanz enthält, zu einem Proben-zuführenden Abschnitt des folgenden Teststreifens;
wobei der Teststreifen eine Membran umfasst, bestehend aus einem porösen Körper, ausgestattet mit wenigstens einem Proben-zuführenden Abschnitt, einem Verteilungsabschnitt und einem Detektionsabschnitt, wobei ein Konjugat, welches einen ersten Antikörper enthält, der mit einer Markierungssubstanz markiert ist, in einem Teil des Verteilungsabschnitts in lösbarer Weise gehalten wird, und der Detektionsabschnitt, in welchem ein zweiter Antikörper immobilisiert ist, in einem Teil des Verteilungsabschnitts auf der abstromigen Seite relativ zu dem das Konjugat haltenden Teil bereitgestellt ist, wobei irgendeiner von dem ersten Antikörper und dem zweiten Antikörper ein Anti-Analyten-Antikörper ist und der andere ein an den Analyten bindender Antikörper ist, und wobei der an den Analyten bindende Antikörper irgendein an den Analyten bindender Antikörper und irgendein an ein dem Analyten ähnliches Antigen bindender Antikörper sein kann;
(B) Inkontaktbringen des Analyten in der Probe mit dem Konjugat in der Gegenwart einer Pufferlösung mit einem pH-Wert von 5,0 bis 6,7; und
(C) Detektieren eines Komplexes des Analyten in der Probe und des Konjugats in dem Detektionsabschnitt;
wobei der Analyt onkofetales Fibronektin ist und die dem Analyten ähnliche Substanz Plasmafibronektin ist.

14. Verfahren nach Anspruch 13, wobei der erste Antikörper ein monoklonaler Anti-Fibronektin-Antikörper ist und der zweite Antikörper ein monoklonaler Anti-onkofetales-Fibronektin-Antikörper ist.

15. Verfahren nach irgendeinem der Ansprüche 13 bis 14, wobei die Pufferlösung irgendeine oder mehrere ist, ausgewählt aus der Gruppe, bestehend aus Phosphat, ADA, MES, ACES, Bis-Tris, Citrat, HEPES, MOPS, PIPES und Tris-Pufferlösung.

16. Verteilungslösung für die Immunchromatographie zum Detektieren von onkofetalem Fibronektin, wobei die Verteilungslösung die folgenden (1) und (2) umfasst:
(1) eine Pufferlösung mit einem pH-Wert von 5,0 bis 6,7, und
(2) ein Konjugat, welches einen ersten mit einer Markierungssubstanz markierten Antikörper enthält.

17. Verwendung einer Probenextraktionslösung, umfassend eine Pufferlösung mit einem pH-Wert von 5,0 bis 6,7, zum Detektieren von onkofetalem Fibronektin.

## Revendications

1. Procédé de détection utilisant l'immunochromatographie, comprenant les étapes (A) à (C) consistant à :
(A) fournir un échantillon contenant éventuellement un analyte et une substance similaire à l'analyte à une portion de fourniture d'échantillon de la bande d'essai suivante ;
la bande d'essai comprenant une membrane constituée par un corps poreux équipé d'au moins la portion de fourniture d'échantillon, une portion de diffusion, et une portion de détection, dans lequel un conjugué contenant un premier anticorps marqué d'une substance de marquage est conservé dans une partie de la portion de diffusion de manière dissoluble, et la portion de détection dans laquelle un second anticorps est immobilisé est pourvue dans une partie de la portion de diffusion sur le côté aval par rapport à la partie de conservation de conjugué, dans lequel l'un quelconque du premier anticorps et du second anticorps est un anticorps anti-analyte et l'autre est un anticorps se liant à l'analyte, et dans lequel l'anticorps se liant à l'analyte peut être tout anticorps se liant à l'analyte et tout anticorps se liant à un antigène similaire à l'analyte ;
(B) amener l'analyte dans l'échantillon en contact avec le conjugué en présence d'une solution tampon de pH 5,0 à pH 6,7 ; et
(C) détecter un complexe de l'analyte dans l'échantillon et le conjugué dans la portion de détection ;
dans lequel l'analyte est une fibronectine oncofœtale, et la substance similaire à l'analyte est une fibronectine plasmatique.

2. Procédé de détection selon la revendication 1, dans lequel le premier anticorps est un anticorps monoclonal anti-fibronectine, et le second anticorps est un anticorps monoclonal anti-fibronectine oncofœtale.

3. Procédé de détection selon l'une quelconque des revendications 1 à 2, dans lequel la solution tampon est l'une ou plusieurs quelconques sélectionnées à partir du groupe constitué par une solution tampon au phosphate, ADA, MES, ACES, Bis-Tris, citrate, HEPES, MOPS, PIPES, et Tris.

4. Bande d'essai immunochromatographique comprenant les composants (1) et (2) suivants :
(1) une membrane constituée par un corps poreux équipé d'au moins une portion de fourniture d'échantillon, une portion de diffusion, et une portion de détection, dans laquelle un conjugué contenant un premier anticorps marqué d'une substance de marquage est conservé dans une partie de la portion de diffusion de manière dissoluble, et la portion de détection dans laquelle un second anticorps est immobilisé est pourvue dans une partie de la portion de diffusion sur le côté aval par rapport à la partie de conservation de conjugué, dans lequel l'un quelconque du premier anticorps et du second anticorps est un anticorps anti-analyte et l'autre est un anticorps se liant à l'analyte, et dans lequel l'anticorps se liant à l'analyte peut être tout anticorps se liant à l'analyte et tout anticorps se liant à un antigène similaire à l'analyte ; et
(2) un composant de solution tampon de pH 5,0 à pH 6,7 qui est contenu dans au moins une partie d'une portion depuis la portion de fourniture d'échantillon à la portion de détection de la portion de diffusion ;
dans laquelle l'analyte est une fibronectine oncofœtale et la substance similaire à l'analyte est une fibronectine plasmatique.

5. Bande d'essai selon la revendication 4, dans laquelle un tampon échantillon, un tampon conjugué, et une membrane immobilisée par un anticorps sont agencés dans cet ordre depuis l'amont, le tampon échantillon inclut la portion de fourniture d'échantillon, le tampon conjugué inclut la partie de conservation de conjugué et le composant de solution tampon, et la membrane immobilisée par un anticorps inclut la portion de détection.

6. Bande d'essai selon la revendication 4 ou 5, dans laquelle l'un quelconque du premier anticorps et du second anticorps est un anticorps monoclonal anti-fibronectine, et l'autre est un anticorps monoclonal anti-fibronectine oncofœtale.

7. Bande d'essai selon la revendication 6, dans laquelle le premier anticorps est un anticorps monoclonal anti-fibronectine, et le second anticorps est un anticorps monoclonal anti-fibronectine oncofœtale.

8. Bande d'essai selon l'une quelconque des revendications 5 à 7, dans laquelle la solution tampon est l'une ou plusieurs quelconques sélectionnées à partir du groupe constitué par une solution tampon au phosphate, ADA, MES, ACES, Bis-Tris, citrate, HEPES, MOPS, PIPES, et Tris.

9. Kit de détection immunochromatographique comprenant les composants (1) et (2) suivants :
(1) une bande d'essai immunochromatographique comprenant une membrane constituée par un corps poreux équipé d'au moins une portion de fourniture d'échantillon, une portion de diffusion, et une portion de détection, dans lequel un conjugué contenant un premier anticorps marqué d'une substance de marquage est conservé dans une partie de la portion de diffusion de manière dissoluble, et la portion de détection dans laquelle un second anticorps est immobilisé est pourvue dans une partie de la portion de diffusion sur le côté aval par rapport à la partie de conservation de conjugué, dans lequel l'un quelconque du premier anticorps et du second anticorps est un anticorps anti-analyte et l'autre est un anticorps se liant à l'analyte, et dans lequel l'anticorps se liant à l'analyte peut être tout anticorps se liant à l'analyte et tout anticorps se liant à un antigène similaire à l'analyte ; et
(2) une solution tampon de pH 5,0 à pH 6,7 ;
dans lequel l'analyte est une fibronectine oncofœtale et la substance similaire à l'analyte est une fibronectine plasmatique.

10. Kit de détection selon la revendication 9, dans lequel l'un quelconque du premier anticorps et du second anticorps est un anticorps monoclonal anti-fibronectine, et l'autre est un anticorps monoclonal anti-fibronectine oncofœtale.

11. Kit de détection selon la revendication 9 ou 10, dans lequel la solution tampon est utilisée comme une solution de diffusion ou une solution d'extraction de prélèvement.

12. Kit de détection selon l'une quelconque des revendications 9 à 11, dans lequel la solution tampon est l'une ou plusieurs quelconques sélectionnées à partir du groupe constitué par une solution tampon au phosphate, ADA, MES, ACES, Bis-Tris, citrate, HEPES, MOPS, PIPES, et Tris.

13. Procédé de réduction de réaction croisée entre un analyte et une substance similaire à l'analyte, dans un procédé de détection d'un analyte dans un échantillon utilisant une bande d'essai immunochromatographique,
le procédé de réduction de réaction croisée comprenant les étapes suivantes consistant à :
(A) fournir un échantillon contenant éventuellement l'analyte et la substance similaire à l'analyte à une portion de fourniture d'échantillon de la bande d'essai suivante ;
la bande d'essai comprenant une membrane constituée par un corps poreux équipé d'au moins une portion de fourniture d'échantillon, une portion de diffusion, et une portion de détection, dans lequel un conjugué contenant un premier anticorps marqué d'une substance de marquage est conservé dans une partie de la portion de diffusion de manière dissoluble, et la portion de détection dans laquelle un second anticorps est immobilisé est pourvue dans une partie de la portion de diffusion sur le côté aval par rapport à la partie de conservation de conjugué, dans lequel l'un quelconque du premier anticorps et du second anticorps est un anticorps anti-analyte et l'autre est un anticorps se liant à l'analyte, et dans lequel l'anticorps se liant à l'analyte peut être tout anticorps se liant à l'analyte et tout anticorps se liant à un antigène similaire à l'analyte ;
(B) amener l'analyte dans l'échantillon en contact avec le conjugué en présence d'une solution tampon de pH 5,0 à pH 6,7 ; et
(C) détecter un complexe de l'analyte dans l'échantillon et le conjugué dans la portion de détection ;
dans lequel l'analyte est une fibronectine oncofœtale, et la substance similaire à l'analyte est une fibronectine plasmatique.

14. Procédé selon la revendication 13, dans lequel le premier anticorps est un anticorps monoclonal anti-fibronectine, et le second anticorps est un anticorps monoclonal anti-fibronectine oncofœtale.

15. Procédé selon l'une quelconque des revendications 13 à 14, dans lequel la solution tampon est l'une ou plusieurs quelconques sélectionnées à partir du groupe constitué par une solution tampon au phosphate, ADA, MES, ACES, Bis-Tris, citrate, HEPES, MOPS, PIPES, et Tris.

16. Solution de diffusion pour une immunochromatographie pour détecter une fibronectine oncofœtale, la solution de diffusion comprenant les éléments (1) et (2) suivants :
(1) une solution tampon de pH 5,0 à pH 6,7, et
(2) un conjugué contenant un premier anticorps marqué d'une substance de marquage.

17. Utilisation d'une solution d'extraction de prélèvement comprenant une solution tampon de pH 5,0 à pH 6,7 pour détecter une fibronectine oncofœtale.
